(19) Europäisches Patentamt
European Patent Office
Office européen des brevets

(11) Numéro de publication: **0 480 026 B1**

(12) # FASCICULE DE BREVET EUROPEEN

(45) Date de publication de fascicule du brevet: **04.01.95**

(51) Int. Cl.$^6$: **B01J 29/08**, C10G 11/05

(21) Numéro de dépôt: **91918432.5**

(22) Date de dépôt: **08.04.91**

(86) Numéro de dépôt internationale :
**PCT/FR91/00286**

(87) Numéro de publication internationale :
**WO 91/15294 (17.10.91 91/24)**

(54) **CATALYSEUR ZEOLITIOUE DE STRUCTURE HEXAGONALE ET SON APPLICATION.**

(30) Priorité: **09.04.90 FR 9004501**

(43) Date de publication de la demande:
**15.04.92 Bulletin 92/16**

(45) Mention de la délivrance du brevet:
**04.01.95 Bulletin 95/01**

(84) Etats contractants désignés:
**AT BE CH DE DK ES FR GB GR IT LI LU NL SE**

(56) Documents cités:
**EP-A- 0 014 291**
**EP-A- 0 023 802**
**EP-A- 0 069 594**
**EP-A- 0 364 352**
**US-A- 3 972 983**
**US-A-03 415 736**

(73) Titulaire: **ELF AOUITAINE**
**Tour Elf,**
**2, Place de la Coupole,**
**La Défense 6**
**F-92400 Courbevoie (FR)**

(72) Inventeur: **DES COURIERES, Thierry**
**152, bd Yves-Farge**
**F-69007 Lyon (FR)**
Inventeur: **GUTH, Jean-Louis**
**59, rue Bellevue**
**F-68200 Brunstatt (FR)**
Inventeur: **PATARIN, Joel**
**13, rue Eugène-Delacroix**
**F-68093 Mulhouse (FR)**
Inventeur: **ZIVKOV, Catherine**
**Résidence l'Arrieulat,**
**13, rue du Gave**
**F-64300 Orthez (FR)**

(74) Mandataire: **Boillot, Marc**
**ELF AOUITAINE**
**Division Propriété Industrielle**
**Tour Elf**
**F-92078 Paris la Défense Cédex 45 (FR)**

**EP 0 480 026 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention concerne un catalyseur à base d'une zéolithe de structure hexagonale apparentée à la faujasite et de haut rapport Si:Al. Elle se rapporte encore à l'application dudit catalyseur aux réactions de catalyse acide réalisées sur des charges hydrocarbonées.

Les zéolithes ont de nombreuses applications industrielles. Elles sont utilisées en particulier dans le domaine des adsorbants, des lessives ou de la catalyse industrielle. Dans ce dernier domaine, l'essentiel des applications telles que le craquage catalytique des distillats sous vide ou des résidus en essence ou encore l'hydrocraquage des distillats sous vide en gazole est satisfait par des catalyseurs renfermant une zéolithe de type faujasite dénommée zéolithe Y et possédant un rapport Si:Al de synthèse compris entre 1,5 et 3.

La stabilité thermique et hydrothermique de cette zéolithe et par conséquent des catalyseurs qui en contiennent reste faible et il est nécessaire de soumettre ladite zéolithe à des traitements post-synthèse pour améliorer ladite stabilité et plus généralement les performances desdits catalyseurs. Pour obtenir une augmentation de la stabilité thermique et hydrothermique de ladite zéolithe, il convient d'augmenter son rapport Si:Al de réseau et dans ce but on peut faire appel à divers traitements parmi lesquels on peut mentionner les techniques telles que traitement à la vapeur d'eau suivi ou non d'un lavage à l'acide des débris aluminiques (US-A-3493519 ; US-A-3506400), traitement par l'acide éthylène diamine tétracétique (G.T.KERR, J. Phys. Chem., 72 (1968), p.2594), traitement par $SiCl_4$ (H.K. BEYER, I.M BELENYKAJA, F. HANGE, M. TIELEN, O.J. GROBET et P.A. JACOBS, J. Chem. Soc., Farad. Trans.1, 81 (1985), pages 2889 à 2901) ou encore traitement au moyen de fluosilicate d'ammonium $(NH_4)_2SiF_6$ (US-A-4503023).

Tous ces traitements sont onéreux, ce qui conduit à une augmentation du coût du catalyseur final. De plus, de tels traitements engendrent des modifications structurales des zéolithes traitées avec, comme conséquence, l'apparition de défauts pouvant pénaliser les performances des catalyseurs incluant ces zéolithes traitées.

La citation US-A-3972983 décrit une zéolithe désignée par le vocable ZSM-20, qui ressemble à la faujasite par certains aspects structuraux. Cette zéolithe présente une structure cristalline en partie hexagonale et un rapport Si:Al d'au moins 3,5 et trouve une utilisation, après échange par des cations hydrogènes ou métalliques et calcination, dans la production de catalyseurs de conversion d'hydrocarbures.

La demande de brevet européen N° 89402788.7 du 10.10.89, publication EP-A-0364352 du 18.04.90, décrit, entre autres, des zéolithes de structure hexagonale apparentées à la faujasite et de rapport Si:Al de synthèse supérieur à 3, qui sont utilisables pour la production de catalyseurs pour les réactions de conversion catalytique de composés organiques et notamment de composés hydrocarbonés.

On a trouvé que certaines de ces zéolithes de structure hexagonale apparentées à la faujasite et de rapport Si:Al de synthèse supérieur à 3 pouvaient être à la base d'excellents catalyseurs pour les réactions de craquage, d'hydrocraquage, d'isomérisation ou encore de dismutation, ces zéolithes particulières présentant, entre autres, une densité élevée de sites acides de force moyenne ou grande et une excellente stabilité thermique et hydrothermique et pouvant également subir, avec beaucoup moins d'inconvénients que la zéolithe Y du fait de leur rapport Si:Al de réseau plus élevé, les traitements postsynthèse éventuels destinés, si besoin est, à augmenter ledit rapport.

L'invention propose donc un catalyseur, qui est du type comportant, en poids, y % d'un liant inorganique et z % d'une zéolithe de structure de symétrie hexagonale apparentée à la faujasite, y et z étant des nombres tels que $1 \leqq y \leqq 99$ et $1 \leqq z \leqq 99$ avec $y + z = 100$, ladite zéolithe présentant des paramètres a, b et c de sa maille hexagonale tels que 1,72 nm $\leqq a = b \leqq$ 1,75 nm et 2,80 nm $\leqq c \leqq$ 2,86 nm et répondant à une formule qui, ramenée à une maille hexagonale, s'écrit

$$(uM_1q^+)\ (vH^+)\ [(SiO_2)_{96-x}(AlO_2)_x]^{x-}\ (t\ H_2O)$$

avec dans cette formule x désignant un nombre tel que $3 < x \leqq 24$, $M_1q^+$ désignant des cations d'au moins un métal de valence q choisi parmi les métaux alcalins et les métaux alcalinoterreux, et u, v, x et t représentant des nombres tels que $t \geqq 0$ selon le degré d'hydratation de la zéolithe, $t = 0$ pour une zéolithe complètement anhydre, et que $qu + v \geqq x$, ledit catalyseur se caractérisant en ce que le nombre v de cation $H^+$ dans la formule de la zéolithe est tel que $0{,}5x \leqq v \leqq x$, de préférence $0{,}8x \leqq v \leqq x$.

Dans le catalyseur selon l'invention, les pourcentages pondéraux y de liant inorganique et z de zéolithe sont de préférence tels que $4 \leqq y \leqq 96$ et $4 \leqq z \leqq 96$ avec $y + z = 100$.

En plus du liant et de la zéolithe, le catalyseur peut encore renfermer, en poids de l'ensemble liant et zéolithe, jusqu'à 15 % et de préférence jusqu'à 10 % d'au moins un métal catalytiquement actif, présent à l'état élémentaire ou sous la forme d'un composé métallique et choisi parmi les métaux nobles tels que

palladium, platine, rhodium et iridium, les métaux de transition non nobles tels que nickel, cobalt, molybdène et tungstène, et certains autres métaux tels que gallium.

Dans la formule de la zéolithe, $M_1q^+$ représente en particulier des cations d'au moins un élément choisi parmi Na, K, Li, Cs, Ca, Sr et Ba.

Un procédé de préparation de la zéolithe utilisable pour la production du catalyseur passe par la formation de la forme alcaline de la zéolithe, c'est-à-dire de la forme pour laquelle les cations de la zéolithe sont des cations alcalins, puis on transforme cette forme alcaline en la forme protonée.

La forme alcaline de la zéolithe à structure de symétrie hexagonale et rapport Si:Al supérieur à 3 peut être obtenue en faisant appel, notamment, à un procédé, décrit dans EP-A-0364352, qui consiste à réaliser tout d'abord un mélange réactionnel ayant un pH supérieur à 10 et renfermant de l'eau, une source de silicium tétravalent, une source d'aluminium trivalent, une source d'ions hydroxydes sous la forme d'une base forte et un agent structurant ST consistant en au moins un macrocycle dont le cycle contient au moins 18 atomes et renferme des hétéroatomes choisis parmi l'oxygène, l'azote, le silicium et le soufre, de manière à obtenir un gel d'aluminosilicate ayant la composition voulue pour permettre sa cristallisation en la zéolithe désirée, puis à maintenir le gel obtenu, éventuellement après mûrissement préalable, à une température au plus égale à 150 °C et sous une pression au moins égale à la pression autogène du mélange constitué par ledit gel pendant une durée suffisante pour effectuer la cristallisation de ce gel en un précurseur de la zéolithe alcaline consistant en ladite zéolithe emprisonnant l'agent structurant ST dans ses cavités et à soumettre ledit précurseur à une calcination pour détruire l'agent structurant et produire la zéolithe alcaline.

Avantageusement la quantité d'agent structurant ST présente dans le mélange réactionnel destiné à former le gel est telle que le rapport molaire $ST:Al^{III}$ aille de 0,1 à 4, ledit rapport allant de préférence de 0,1 à 1 et tout particulièrement de 0,2 à 0,5.

En particulier, les ingrédients constituant le mélange réactionnel donnant naissance au gel d'aluminosilicate sont utilisés de telle sorte que ledit gel ait, en termes de rapports molaires, la composition suivante

| | Intervalles avantageux | Intervalles préférés |
|---|---|---|
| $Si^{IV} : Al^{III}$ | 2 à 20 | 4 à 10 |
| $OH^- : Al^{III}$ | 0,5 à 8 | 1 à 6 |
| $ST : Al^{III}$ | 0,1 à 4 | 0,1 à 1 |
| $H_2O : Al^{III}$ | 40 à 200 | 50 à 150 |

Les agents structurants ST utilisables pour constituer le mélange réactionnel donnant naissance au gel d'aluminosilicate peuvent être notamment

- des éthers couronnes dont le cycle renferme 18 à 24 atomes et comporte uniquement des atomes d'oxygène comme hétéroatomes, parmi lesquels on peut citer les composés suivants :
  1, 4, 7, 10, 13, 16-hexaoxacyclooctadécane (éther couronne "18 crown 6"),
  2, 3, 11, 12-dibenzo 1, 4, 7, 10, 13, 16-hexaoxacyclooctadécane (éther couronne "dibenzo 18 crown 6"),
  2, 3, 11, 12-dicyclohexano 1, 4, 7, 10, 13, 16-hexaoxacyclooctadécane (éther couronne "dicyclohexano 18 crown 6"),
  2, 3, 14, 15-dibenzo 1, 4, 7, 10, 13, 16, 19, 22-octaoxacyclotétracosane (éther couronne "dibenzo 24 crown 8"),
  2, 3, 14, 15-dicyclohexano 1, 4, 7, 10, 13, 16, 19, 22-octaoxacyclotétracosane (éther couronne "dicyclohexano 24 crown 8"),
- des composés ayant une structure comparable à celle des éthers couronnes ci-dessus mais dont les atomes d'oxygène du cycle sont remplacés en partie ou en totalité par des substituants choisis parmi les atomes de soufre et les groupement >NH, >NR et

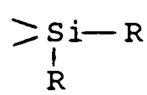

  dans lesquels R est un radical hydrocarbyle en $C_1$ à $C_4$, parmi lesquels on peut citer les composés suivants :
  1, 4 , 7, 10, 13, 16-hexaazacyclooctadécane trisulfate (hexacyclène trisulfate),
  20-(diméthyl-1,1 sila) 1, 4, 7, 10, 13, 16, 19-heptaoxacycloeicosane (éther couronne "diméthylsila

20 crown 7"),

1, 4, 7, 10, 13, 16-hexathiacyclooctadécane,

1, 7, 10, 16-tétraoxa 4, 13-diazacyclooctadécane (Kryptofix 22).

Parmi les sources de silicium tétravalent $Si^{IV}$ utilisables dans la préparation du mélange réactionnel destiné à former le gel d'aluminosilicate, on peut citer les silices solides finement divisées sous la forme d'hydrogels, d'aérogels ou de suspensions colloidales, les silicates hydrosolubles tels que les silicates alcalins comme le silicate de sodium, les esters siliciques hydrolysables tels que les orthosilicates de tétraalcoyle de formule $Si(OR)_4$ dans laquelle R est un alcoyle en $C_1$ à $C_4$ tel que méthyle ou éthyle. La source de silicium est mise en oeuvre sous la forme d'une solution aqueuse vraie, cas des silicates hydrosolubles, ou bien d'une suspension aqueuse qui peut être colloidale, cas des silices finement divisées.

Conviennent comme sources d'aluminium trivalent $Al^{III}$ les sels d'aluminium tels que sulfate, nitrate, chlorure, fluorure, acétate, les oxydes et hydroxyoxydes d'aluminium, les aluminates et notamment les aluminates alcalins tels que l'aluminate de sodium, les esters d'aluminium tels que les aluminium trialcoxydes de formule $Al(OR)_3$ dans laquelle R désigne un radical alcoyle en $C_1$ à $C_4$ tel que méthyle, éthyle ou propyle.

La source d'ions hydroxydes est choisie notamment parmi les hydroxydes des métaux alcalins, les hydroxydes des métaux alcalino-terreux Ca, Sr et Ba et les bases fortes organiques, notamment hydroxydes d'ammoniums quaternaires, la préférence allant aux bases minérales et notamment à la soude NaOH.

Le mélange des ingrédients constituant le milieu réactionnel destiné à former le gel d'aluminosilicate peut être réalisé dans un ordre quelconque. Avantageusement on effectue ledit mélange en préparant tout d'abord, à température ambiante, une solution aqueuse basique renfermant une base forte et l'agent structurant, puis en incorporant à cette solution une solution aqueuse de la source d'aluminium trivalent et une solution aqueuse ou une suspension, colloidale ou non, de la source de silicium tétravalent.

Le pH du mélange réactionnel, dont la valeur est supérieure à 10, est de préférence proche de 13.

Avant de procéder à la cristallisation du gel, on peut ajouter au milieu réactionnel destiné à former ledit gel, des germes de cristallisation en quantité allant avantageusement de 0, 1 à 10 % en poids du milieu réactionnel. Ces germes peuvent être produits soit par broyage d'une zéolithe de même nature que la phase cristalline à produire ou encore par synthèse à partir d'une solution de nucléation appropriée. Une telle solution a par exemple la composition suivante, exprimée en oxydes :

15 $Na_2O$ ; 1 $Al_2O_3$ ; 10 $SiO_2$ ; 18O $H_2O$.

En l'absence d'ajout de germes, il est avantageux de soumettre le gel d'aluminosilicate, formé à partir du mélange réactionnel, à un mûrissement, dans une enceinte fermée, à une température inférieure à la température de cristallisation pendant une durée pouvant aller d'environ 6 heures à environ 6 jours. Ledit mûrissement peut être réalisé en statique ou sous agitation.

La cristallisation du gel d'aluminosilicate, avec ou sans germe, s'effectue en chauffant le mélange réactionnel à une température au plus égale à 150°C et de préférence allant de 90°C à 120°C et sous une pression correspondant au moins à la pression autogène du mélange réactionnel formant le gel. La durée du chauffage nécessaire à la cristallisation se situe généralement entre 2 heures et quinze jours.

Les cristaux obtenus, qui consistent en la zéolithe alcaline emprisonnant l'agent structurant dans ses pores et cavités, sont séparés du milieu de cristallisation par filtration, puis lavés à l'eau distillée ou désionisée jusqu'à obtenir des eaux de lavage dont le pH est inférieur à 9. Les cristaux lavés sont ensuite séchés en étuve à une température comprise entre 50°C et 100°C et de préférence aux environs de 70°C.

La zéolithe alcaline est obtenue à partir des cristaux séchés en soumettant lesdits cristaux à une calcination à une température supérieure à 300°C et de préférence comprise entre 400°C et 700°C pendant une durée suffisante pour éliminer l'agent structurant qu'ils renferment.

La forme protonée (acide) de la zéolithe est préparée, à partir de la forme alcaline obtenue comme décrit ci-dessus, en effectuant tout d'abord une mise en contact de la zéolithe alcaline calcinée avec une solution aqueuse d'un sel d'ammonium en opérant à des températures allant, par exemple, de 50°C à 100°C pour réaliser un échange des cations de métal alcalin ou alcalino-terreux de la zéolithe alcaline par des cations $NH_4^+$, l'opération étant répétée plusieurs fois si besoin est, puis en soumettant la zéolithe renfermant des cations $NH_4^+$ à l'action de températures appropriées pour effectuer une dégradation thermique des cations $NH_4^+$ et obtenir la forme acide de la zéolithe, c'est-à-dire la forme pour laquelle, dans la formule donnée plus haut, tous les cations $M^{n+}$ sont des cations $H^+$.

Le procédé de synthèse décrit plus haut conduit à des zéolithes dont le rapport Si:Al de réseau est égal ou supérieur à 3. Si besoin est, on peut encore augmenter ledit rapport Si:Al en soumettant la zéolithe,

dans sa forme alcaline ou acide ou échangée, à l'un des traitements de désalumination connus à cet effet et, par exemple, à l'un des traitements à la vapeur d'eau, à l'acide éthylènediaminetétracétique, par $SiCl_4$ ou par $(NH_4)_2SiF_6$ cités précédemment.

Comme indiqué plus haut, les zéolithes utilisées dans le catalyseur selon l'invention sont des zéolithes qui sont apparentées à la faujasite et présentent une structure de symétrie hexagonale et un rapport Si:Al de réseau allant de 3 à 31 et de préférence de 3 à 20. Ces zéolithes ont des paramètres a, b et c de la maille hexagonale tels que a = b et que 1,72nm $\leqq$ a $\leqq$ 1,75 nm et 2,80 nm $\leqq$ c $\leqq$ 2,86 nm et présentent, après calcination à 600 °C pendant 4 heures, un diagramme de diffraction des rayons X comparable à celui du tableau I ci-après.

Le rapport Si:Al de la zéolithe peut être déterminé par analyse chimique, ou bien par radiocristallographie (D.W.BRECK:"Zeolite Molecular Sieves", Ed. John Wiley and Sons, New York, 1974, page 94) ou encore par RMN du silicium 29 (J. KLINOWSKI : "Progress in NMR Spectroscopy", 1984, Vol.16, pages 237 à 309).

Le diagramme de diffraction des rayons X est obtenu au moyen d'un diffractomètre en utilisant la méthode classique des poudres avec le rayonnement $K_\alpha$ du cuivre. Un étalon interne permet de déterminer précisément les valeurs des angles $2\theta$ associés aux pics de diffraction. Les différentes distances interréticulaires $d_{hkl}$ , caractéristiques de l'échantillon, sont calculées à partir de la relation de BRAGG. L'intensité relative I:Io affectée à chaque valeur $d_{hkl}$ est estimée à partir de la hauteur du pic de diffraction correspondant. Pour caractériser cette intensité relative on utilise une échelle de symbole comme suit : FF = très forte, F = forte, mF = moyennement forte, m = moyenne, mf = moyennement faible, f = faible et ff = très faible.

Dans la colonne des $d_{hkl}$ du tableau I, on a donné les valeurs moyennes des distances interréticulaires. Chacune de ces valeurs doit être affectée de l'erreur de mesure $\Delta(d_{hkl})$ comprise en ± 0,2 et ± 0,008. Les variations qui peuvent être observées par rapport à ces valeurs moyennes sont essentiellement liées à la nature des cations de compensation et au rapport Si:Al de la zéolithe. Les mêmes remarques s'appliquent aux intensités relatives I/Io.

TABLEAU I

| 2 θ (°) | $d_{hkl}$ ($10^{-1}$nm) | (h k l) | I/Io |
|---|---|---|---|
| 5,88 | 15,03 ± 0,2 | (1 0 0) | FF |
| 6,23 | 14,2 | (0 0 2) | FF |
| 6,66 | 13,3 | (1 0 1) | F |
| 8,40 | 10,52 | (1 0 2) | f |
| 10,19 | 8,68 ± 0,08 | (1 1 0) | F |
| 11,06 | 7,99 | (1 0 3) | mF |
| 11,78 | 7,51 | (2 0 0) | mF |
| 11,95 | 7,40 | (1 1 2) | mF |
| 13,49 | 6,56 | (2 0 2) | ff |
| 15,06 | 5,88 ± 0,05 | (2 0 3) | f |
| 15,58 | 5,68 | (0 0 5) | F |
| 15,89 | 5,57 | (2 1 1) | f |
| 16,73 | 5,29 | (1 0 5) | f |
| 17,18 | 5,16 | (2 0 4) | F |
| 18,22 | 4,87 | (2 1 3) | f |
| 18,79 | 4,72 | (1 1 5) | f |
| 19,67 | 4,51 | (1 0 6) | f |
| 20,45 | 4,34 | (2 2 0) | mF |
| 22,22 | 4,00 | (3 1 2) | f |
| 22,75 | 3,91 | (1 0 7) | f |
| 23,30 | 3,82 | (3 1 3) | f |
| 23,66 | 3,76 | (3 0 5) | F |
| 24,75 | 3,59 | (3 1 4) | f |
| 25,83 | 3,45 | (2 2 5) | f |
| 26,52 | 3,36 | (2 1 5) | f |
| 27,16 | 3,28 | (4 1 0) | f |
| 28,77 | 3,103 ±0,008 | (3 2 4) | f |
| 30,38 | 2,942 | (4 0 6) | ff |
| 30,89 | 2,894 | (3 3 0) | f |
| 31,20 | 2,866 | (5 0 3) | f |
| 31,56 | 2,834 | (3 3 2) | ff |

Le liant inorganique que l'on associe à la zéolithe pour former le catalyseur selon l'invention peut être inerte, c'est-à-dire sans action catalytique dans la réaction pour laquelle le catalyseur est utilisé, ou bien être actif, c'est-à-dire présenter une certaine activité catalytique pour la réaction considérée. Le liant inorganique peut consister notamment en un ou plusieurs oxydes réfractaires comme, par exemple, alumine, silice, silice/alumine, argile, magnésie. Le liant inorganique peut également consister en un catalyseur présentant une activité pour une réaction et auquel l'addition d'une zéolithe selon l'invention améliore substantiellement ladite activité ou apporte une activité catalytique pour une autre réaction.

Le catalyseur selon l'invention peut être préparé à partir du liant inorganique et de la zéolithe en faisant appel aux diverses techniques proposées à cet effet. On peut en particulier faire appel à une technique d'extrusion consistant à préparer une pâte à partir de quantités appropriées du liant inorganique et de la zéolithe et d'une petite quantité d'eau, puis à soumettre la pâte ainsi formée à une extrusion à travers une filière pour former des granulés. On peut encore préparer le catalyseur sous la forme de microsphères par séchage par atomisation d'une suspension aqueuse du liant inorganique et de la zéolithe. On peut également mettre en forme le catalyseur à partir du liant inorganique et de la zéolithe en utilisant une technique de pastillage ou de dragéification.

La mise en forme du catalyseur peut être encore effectuée à partir du liant inorganique et de la forme alcaline de la zéolithe, après quoi le produit résultant de cette mise en forme est soumis aux opérations décrites précédemment pour obtenir la forme de la zéolithe renfermant des cations $H^+$.

Lorsque le catalyseur selon l'invention renferme un métal ou un composé métallique en plus des cations de compensation de la zéolithe, ledit métal ou composé métallique est incorporé au catalyseur par toute méthode connue, par exemple, par addition au mélange du liant inorganique et de la zéolithe avant la mise en forme dudit mélange, par imprégnation du liant inorganique et/ou de la zéolithe avant mélange de

ces derniers ou encore par imprégnation du produit résultant de la mise en forme du mélange du liant inorganique et de la zéolithe.

Le catalyseur selon l'invention est utilisable pour promouvoir les réactions de catalyse acide réalisées sur des charges hydrocarbonées.

En particulier ledit catalyseur convient bien pour le craquage catalytique de charges hydrocarbonées, notamment craquage catalytique de distillats sous vide ou de résidus en essence, en opérant en particulier en lit fluidisé ("Fluid Catalytic Cracking" abrégé en "FCC") ou en lit mobile à des températures allant, par exemple, de 450 °C à 650 °C.

Le catalyseur selon l'invention convient encore pour promouvoir l'hydrocraquage de charges hydrocarbonées, en particulier hydrocraquage de distillats sous vide en gazole, en opérant à des températures supérieures à 250 °C, notamment entre 350 °C et 500 °C, sous une pression supérieure à 15 bars, avantageusement supérieure à 30 bars et avec une vitesse volumique horaire comprise entre 0,2 et 10.

Le catalyseur précité convient également pour promouvoir l'alkylation des hydrocarbures aromatiques, notamment benzène, par les oléfines, ainsi que l'isomérisation des hydrocarbures alkylaromatiques tels que les xylènes.

La zéolithe présente dans le catalyseur possède une surface spécifique, déterminée par adsorption d'azote selon la méthode B.E.T. simplifiée à un point (norme NF X 11-622), comprise entre 100 $m^2/g$ et 1000 $m^2/g$ et de préférence entre 300 $m^2/g$ et 900 $m^2/g$.

Les exemples suivants sont donnés à titre non limitatif pour illustrer l'invention.

EXEMPLE 1 :

Synthèse d'une zéolithe acide apparentée à la faujasite et présentant une structure de symétrie hexagonale et un rapport Si:Al supérieur à 3.

On préparait tout d'abord un gel d'aluminosilicate en opérant comme suit dans un récipient de capacité appropriée, le contenu dudit récipient étant maintenu sous agitation pendant toute la durée de l'opération.

Dans le récipient on introduisait 9 parties d'eau, puis 0,44 partie de soude NaOH et, après dissolution de la soude, 1,45 partie d'éther couronne "18 crown 6". Après dissolution totale de l'éther couronne, on ajoutait alors au contenu du récipient 1 partie d'un aluminate de sodium renfermant 56% d'$Al_2O_3$ et 37% de $Na_2O$ et chauffait légèrement le mélange réactionnel pour dissoudre complètement l'aluminate. Après retour à température ambiante, on introduisait alors dans le récipient 8,2 parties d'une suspension colloidale de silice renfermant 40% de $SiO_2$ et 60% d'eau.

On obtenait ainsi un gel d'aluminosilicate dont la composition molaire, rapportée à une mole d'$Al_2O_3$, était la suivante :

$10SiO_2$ ; $1Al_2O_3$ ; 2,1 $Na_2O$ ; 1 éther couronne : $140H_2O$

Le gel obtenu était soumis à un mûrissement à température ambiante pendant 24 heures dans un récipient fermé.

Le gel mûri était ensuite placé dans un autoclave et maintenu à 110 °C dans ce dernier pendant 336 heures pour former un produit cristallisé. Les cristaux formés étaient séparés du milieu réactionnel par filtration, puis lavés à l'eau distillée jusqu'à faible basicité (pH inférieur à 9) des eaux de lavage et enfin séchés à environ 60 °C dans une étuve.

Les cristaux séchés étaient ensuite calcinés afin d'éliminer l'éther couronne utilisé comme agent structurant, ladite calcination étant réalisée sous un léger débit d'air égal à 1,5 $l.h^{-1}$ selon le profil thermique suivant

| rampe de température | | durée (mn) |
|---|---|---|
| 20 °C | 100 °C | 30 |
| 100 °C | 100 °C | 60 |
| 100 °C | 200 °C | 30 |
| 200 °C | 200 °C | 60 |
| 200 °C | 450 °C | 60 |
| 450 °C | 450 °C | 180 |
| 450 °C | 25 °C | (inertie du four) |

Le solide obtenu après calcination, qui consiste en la forme alcaline de la zéolithe, présentait un diagramme de diffraction des rayons X comparable à celui du tableau I caractéristique des zéolithes calcinées présentant une structure de symétrie hexagonale et apparentées à la faujasite.
La formule trouvée pour cette zéolithe, ramenée à une maille de la structure hexagonale, s'écrit à l'état anhydre

$19,5\ Na^{+}\ [(SiO_2)_{76,9}\ (AlO_2)_{19,1}]^{19,1-}$

On observe un très léger excès de charge positive par rapport à la neutralité.
Le solide calciné, consistant en la forme sodique calcinée de la zéolithe, était mis en contact avec une solution deux fois molaire de $NH_4NO_3$ pour réaliser un échange des cations $Na^+$ de la zéolithe par des cations $NH_4^+$, les conditions opératoires de l'opération d'échange étant les suivantes :

. volume de liquide par g de solide : 25 ml
. température de l'échange : 80 °C
. durée de l'échange : 1,5 heure

L'opération d'échange était répétée cinq fois.
La forme acide de la zéolithe était ensuite obtenue par dégradation thermique des cations ammonium présents dans la zéolithe soumise à l'échange, ladite dégradation thermique étant réalisée sous un léger débit d'air de 1,5 l/heure selon le profil thermique ci-après :

| rampe de température | | durée (mn) |
|---|---|---|
| 20 °C | 200 °C | 120 |
| 200 °C | 200 °C | 60 |
| 200 °C | 550 °C | 240 |
| 550 °C | 550 °C | 180 |
| 550 °C | 25 °C | (inertie du four) |

La zéolithe acide résultant du traitement thermique ci-dessus était alors placée à l'humidificateur sous une humidité relative de 80% de façon à stabiliser son taux d'hydratation.
La zéolithe acide obtenue avait une structure de symétrie hexagonale et un rapport Si:Al égal à environ 4 et présentait un diagramme de diffraction des rayons X comparable à celui du tableau I, les paramètres a, b et c de la maille hexagonale étant tels que a = b = 1,74nm et c = 2,83 nm. Le taux de fraction cristalline de cette zéolithe acide, déterminée par diffraction des rayons X, était égal à 100%.
La composition chimique pondérale en oxydes de la zéolithe acide réhydratée comme indiqué ci-dessus est la suivante :

| $Na_2O$ | $SiO_2$ | $Al_2O_3$ | $H_2O$ |
|---|---|---|---|
| 0,31% | 60,10% | 12,67% | 26,9% |

La formule trouvée pour cette zéolithe, ramenée à une maille de la structure hexagonale, s'écrit à l'état anhydre (t = o dans la formule générale) :

$(0,8\ Na^{+})\ (18,3\ H^{+})\ [(SiO_2)_{76,9}\ (AlO_2)_{19,1}]^{19,1-}$

<u>EXEMPLE 2</u> :

<u>Etude de l'acidité par thermodésorption programmée d'ammoniac</u>

En faisant appel à la technique de thermodésorption programmée d'ammoniac, on étudiait la répartition des sites acides des trois zéolithes acides suivantes :

A) - zéolithe acide selon l'invention obtenue comme décrit dans l'exemple 1 ;

B) - zéolithe acide de type faujasite (symétrie cubique) obtenue en soumettant une zéolithe Y dans sa forme ammonium $NH_4Y$, qui est commercialisée sous l'appellation ZF 110 par la société ZEOCAT, à un traitement de dégradation thermique des cations $NH_4^+$ identique à celui décrit dans l'exemple 1. Cette zéolithe acide, après réhydratation sous une humidité relative de 80% avait les caractéristiques physico-chimiques données dans le tableau II ci-après.

TABLEAU II

| Composition chimique pondérale en oxydes (%) | | | | Rapport Si : Al mesuré | Fraction cristalline*) (%) |
|---|---|---|---|---|---|
| $Na_2O$ | $SiO_2$ | $Al_2O_3$ | $H_2O$ | | |
| 1,14 | 53,44 | 17,62 | 27,80 | 26 | 90 |

*) La référence utilisée est la zéolithe acide de l'exemple 1.

La formule trouvée pour la zéolithe ZF 110 dans sa forme acide, ramenée à une maille de sa structure cubique, s'écrit à l'état anhydre

$(5,7\ Na^+)\ (47,9\ H^+)\ [(SiO_2)_{138,4}\ (AlO_2)_{53,6}]^{53,6-}$

C) - zéolithe acide de type faujasite (symétrie cubique) consistant en la forme acide d'une zéolithe Y désaluminée commerciale (zéolithe ZF 510 de la société ZEOCAT) sans débris aluminique.
Les caractéristiques physico-chimiques de l'échantillon de cette zéolithe acide après réhydratation sous une humidité relative de 80% sont données dans le tableau III.

TABLEAU III

| Composition chimique pondérale en oxydes (%) | | | | Rapport Si:Al mesuré | Fraction cristalline*) (%) |
|---|---|---|---|---|---|
| $Na_2O$ | $SiO_2$ | $Al_2O_3$ | $H_2O$ | | |
| 0,2 | 66,6 | 6,64 | 26,6 | 8,5 | 95 |

*) La référence utilisée est la zéolithe acide de l'exemple 1.

La formule trouvée pour la zéolithe acide ZF 510, ramenée à une maille de sa structure cubique, s'écrit à l'état anhydre

$(1,0\ Na^+)\ (19,2\ H^+)\ [(SiO_2)_{171,8}\ (AlO_2)_{20,2}]^{20,2-}$

Les opérations successives effectuées au cours de chaque essai de thermodésorption étaient réalisées sous un léger débit de gaz, ces opérations étant schématisées dans le tableau IV

TABLEAU IV

| Opération | Température (°C) | Durée (mn) | Gaz Nature Débit (l/h) | |
|---|---|---|---|---|
| Activation de la zéolithe en deux stades | 200<br>550 | 30<br>60 | Hélium | 1,5 |
| Adsorption de $NH_3$ | 100 | 15 | $NH_3$ | 1,5 |
| Purge de l'excès de $NH_3$ | 140 | 30 | $NH_3$ | 4,0 |
| Désorption de $NH_3$ (un palier de désorption de 60 minutes est réalisé à chaque température | 200<br><br>250<br>300<br>350<br>400<br>450<br>500<br>550 | 60<br><br>60<br>60<br>60<br>60<br>60<br>60<br>60 | Hélium | 1,5 |

A partir du volume de $NH_3$ libéré pour chaque palier de désorption, on déterminait la quantité correspondante de cations $H^+$ et rapportait cette quantité en milliéquivalents par gramme (meq $H^+$/g) de la zéolithe acide soumise à l'essai de thermodésorption.
Les résultats obtenus sont rassemblés dans le tableau V.

TABLEAU V

| Température (°C) | Quantité de $H^+$ dans la zéolithe acide(meq $H^+$/g) | | |
|---|---|---|---|
| | Zéolithe acide A | Zéolithe acide B | Zéolithe acide C |
| 150 | 0,034 | 0,040 | 0,029 |
| 200 | 0,487 | 0,297 | 0,226 |
| 250 | 0,422 | 0,257 | 0,215 |
| 300 | 0,371 | 0,229 | 0,213 |
| 350 | 0,398 | 0,197 | 0,214 |
| 400 | 0,344 | 0,142 | 0,124 |
| 450 | 0,152 | 0,086 | 0,044 |
| 500 | 0,054 | 0,052 | 0,026 |
| 550 | 0,049 | 0,043 | 0,029 |
| Acidité totale | 2,311 | 1,343 | 1,120 |

Au vu des résultats présentés dans le tableau V, il apparaît que la densité de sites acides et le nombre de sites de fortes acidités sont plus importants pour la zéolithe acide A (selon l'invention) que pour les zéolithes acides témoins B et C.

EXEMPLE 3:

Activité catalytique de la zéolithe acide en craquage du n-heptane

On évaluait les activités catalytiques de la zéolithe acide A selon l'invention et de chacune des zéolithes acides témoins B et C de l'exemple 2 en craquage catalytique du n-heptane.
Pour ce faire, on opérait dans un réacteur en quartz ayant un diamètre intérieur de 18mm et équipé en son centre d'une plaque de verre fritté sur laquelle on plaçait 500mg de la zéolithe acide à soumettre à l'essai. Après activation de la zéolithe placée sur la plaque de verre par chauffage à 200°C pendant 30mn puis à 550°C pendant 60mn sous un courant d'azote ayant un débit de 1,5 l/heure, on envoyait sur la zéolithe activée un mélange d'azote et de n-heptane.
Les conditions opératoires étaient les suivantes :

. pression : atmosphérique
. rapport des pressions partielles $p_{N2}$ : $p_{n-heptane}$ : 6,6
. température de la réaction : 350 °C
. (poids de réactif/poids de zéolithe) par heure : 2,4 $h^{-1}$

On donne dans le tableau VI la valeur de l'activité catalytique, exprimée en millimoles de n-heptane craqué par gramme de zéolithe et par heure (mmoles.$h^{-1}$.$g^{-1}$), en fonction du temps de réaction de la zéolithe en minutes (mn).

TABLEAU VI

| Temps de réaction (mn) | n-heptane craqué (mmoles.$h^{-1}$.$g^{-1}$) | | |
|---|---|---|---|
| | Zéolithe acide A | Zéolithe acide B | Zéolithe acide C |
| 8 | 22,90 | 3,61 | 13,88 |
| 18 | 16,69 | 2,69 | 8,31 |
| 28 | 13,07 | 2,25 | 5,67 |
| 38 | 11,00 | 1,88 | 4,72 |
| 48 | 8,83 | 1,55 | 3,90 |
| 68 | 6,53 | 1,29 | 2,47 |
| 125 | 3,10 | 0,64 | 1,17 |

Les résultats ci-dessus font ressortir que la zéolithe acide A selon l'invention présente une activité catalytique supérieure à celle des zéolithes acides témoins B et C.

EXEMPLE 4 :

Stabilité thermique de la zéolithe acide

Des échantillons de la zéolithe acide A (selon l'invention) et des zéolithes acides témoins B et C de l'exemple 2 étaient calcinés à 800 °C pendant 4 heures sous un débit d'air sec de 1,5 l/heure.
Les produits calcinés obtenus étaient réhydratés sous une humidité relative de 80%, après quoi on déterminait leur taux de fraction cristalline par diffraction des rayons X en prenant comme référence la zéolithe acide A (zéolithe acide de l'exemple 1).
Les taux de fraction cristalline des zéolithes A, B et C calcinées comme indiqué ci-dessus étaient respectivement de 90% (A calcinée), 25% (B calcinée) et 110% (C calcinée).

EXEMPLE 5 :

Influence de la stabilité thermique de la zéolithe acide sur ses caractéristiques catalytiques :

Les zéolithes acides A et C calcinées de l'exemple 4, qui ont une cristallinité comparable, étaient soumises à l'essai de thermodésorption programmée de $NH_3$ comme indiqué dans l'exemple 2 et également à l'essai de craquage catalytique du n-heptane comme indiqué dans l'exemple 3.
Les résultats de ces essais sont présentés respectivement dans les tableaux VII et VIII.

TABLEAU VII

| Thermodésorption programmée de $NH_3$ | | |
|---|---|---|
| Température (°C) | Acidité (meq $H^+$/g) | |
| | Zéolithe A calcinée de l'exemple 4 | Zéolithe C calcinée de l'exemple 4 |
| 150 | 0,097 | 0,032 |
| 200 | 0,287 | 0,180 |
| 250 | 0,219 | 0,130 |
| 300 | 0,157 | 0,099 |
| 350 | 0,137 | 0,076 |
| 400 | 0,068 | 0,045 |
| 450 | 0,024 | 0,019 |
| 500 | 0,021 | 0,014 |
| 550 | 0,021 | 0,016 |
| Acidité totale | 1,031 | 0,611 |

TABLEAU VIII

| Temps de réaction (mn) | Craquage du n-heptane | |
|---|---|---|
| | n-heptane craqué (mmoles.$h^{-1}$.$g^{-1}$) | |
| | Zéolithe A calcinée de l'exemple 4 | Zéolithe C calcinée de l'exemple 4 |
| 8 | 10,11 | 3,8 |
| 18 | 6,31 | 2,13 |
| 28 | 4,65 | 1,62 |
| 38 | 3,28 | 1,22 |
| 48 | 2,76 | 0,99 |
| 68 | 1,97 | 0,51 |
| 125 | 0,52 | 0,19 |

La zéolithe acide selon l'invention (zéolithe A) possède encore, après calcination à 800°C sous air, des caractéristiques catalytiques supérieures à celles de la zéolithe témoin C calcinée dans les mêmes conditions.

EXEMPLE 6 :

Stabilité hydrothermique de la zéolithe acide

On déterminait la surface spécifique d'échantillons des zéolithes acides A, B et C de l'exemple 2 et d'une zéolithe acide US Y (zéolithe acide Y ultra-stabilisée) provenant de la société KATALISTIKS (zéolithe acide D), avant et après avoir soumis lesdits échantillons à l'action de 100% de vapeur d'eau à 750°C pendant 2 heures.
La détermination des surfaces spécifiques était effectuée par adsorption d'azote selon la méthode B.E.T. simplifiée à un point.
Les résultats obtenus sont rassemblés dans le tableau IX.

TABLEAU IX

| Zéolithe acide utilisée | Surface spécifique ($m^2/g$) | | Rétention de surface (%) |
|---|---|---|---|
| | Avant traitement à la vapeur d'eau | Après traitement à la vapeur d'eau | |
| A | 632 | 460 | 73 |
| B | 585 | 9 | 1,5 |
| C | 615 | 542 | 88 |
| D | 562 | 432 | 77 |

Le rapport Si:Al de réseau de la zéolithe acide D est d'environ 4.

EXEMPLE 7:

Essai de laboratoire d'évaluation de l'activité catalytique sur charge réelle ("Microactivity test") :

On déterminait l'efficacité de deux catalyseurs A (selon l'invention) et D (comparatif) comme catalyseurs de craquage en lit fluidisé (FCC) en utilisant la méthode de laboratoire ("Microactivity test") définie dans la norme ASTM D 3907-87. La charge hydrocarbonée traitée était une charge réelle prélevée en raffinerie, dont les caractéristiques sont résumées ci-après :

- . $d_4^{15}$ = 0,922
- . point d'aniline = 82,1 °C
- . viscosité à 100 °C = $6{,}08.10^{-6}$ $m^2/s$
- . teneur en soufre = 2,68% en poids
- . teneur en composés azotés = 800 ppm (exprimés en azote)
- . carbone Conradson = 0,44%
- . distillation selon ASTM D 1160

| | |
|---|---|
| 5 % | 324 °C |
| 10 % | 362 °C |
| 50 % | 431 °C |
| 90 % | 502 °C |
| 95 % | 519 °C |

Les catalyseurs A et D utilisés étaient les suivants :

- . Catalyseur A (selon l'invention): obtenu à partir d'un mélange renfermant, en poids, 95% d'un catalyseur E et 5% de la zéolithe acide A de l'exemple 3 (zéolithe acide de l'exemple 1). Le catalyseur E consistait en un catalyseur industriel de craquage catalytique de la société CROSFIELD (catalyseur SLS 100) soumis à un traitement à la vapeur d'eau à 750 °C pendant 17 heures sous 100% de vapeur d'eau.
- . Catalyseur D (comparatif) : obtenu à partir d'un mélange renfermant, en poids, 95% du catalyseur E précité et 5% de la zéolithe acide D.

Les conditions opératoires utilisées dans l'essai étaient telles qu'indiquées ci-après :

- . quantité de catalyseur : 3g
- . température du réacteur : 530 °C
- . rapport pondéral catalyseur : charge : 6
- . (poids de charge/poids de catalyseur)/h : (30g/g)/heure

Les résultats obtenus sont résumés dans le tableau X.

Ces résultats font apparaître que le catalyseur A possède une activité équivalente à celle du catalyseur D. Par ailleurs, le catalyseur A présente une meilleure sélectivité en coke et en essence, les GPL obtenus étant plus riches en oléfines, ce qui laisse présager un indice d'octane plus élevé pour les essences obtenues.

TABLEAU X

| | Catalyseur A | Catalyseur D |
|---|---|---|
| Rendements (% poids) | 8,04 | 8,55 |
| Coke | 0,08 | 0,09 |
| $H_2$ | 1,23 | 1,32 |
| $C_1$ | 2,22 | 2,42 |
| Gas ($H_2 + C_1 + C_2$) | 3,53 | 3,83 |
| $C_3$ | 3,57 | 4,43 |
| $C3^{=}$ | 4,63 | 4,16 |
| iso $C_4$ | 6,88 | 7,18 |
| n-$C_4$ | 2,29 | 2,55 |
| $C_4^{=}$ | 4,84 | 4,38 |
| GPL ($\Sigma C_3 + \Sigma C_4$) | 22,21 | 22,70 |
| Total gaz | 25,74 | 26,53 |
| Essence totale (Point final 220 ° C) | 46,19 | 45,28 |
| Conversion (% poids) (Essence + Gaz + Coke) | 79,97 | 80,36 |
| Gazole (220 ° C-350 ° C) | 13,87 | 13,45 |
| Résidus (>350 ° C) | 6,16 | 6,19 |
| Rapport $C_3^{=}:\Sigma C_3$ | 0,56 | 0,48 |
| Rapport $C_4^{=}:\Sigma C_4$ | 0,35 | 0,31 |

**Revendications**

1. Catalyseur comportant, en poids, y% d'un liant inorganique et z% d'une zéolithe de structure de symétrie hexagonale apparentée à la faujasite, y et z étant des nombres tels que $1 \leqq y \leqq 99$ et $1 \leqq z \leqq 99$ avec $y + z = 100$, ladite zéolithe présentant des paramètres a, b et c de sa maille hexagonale tels que $1,72nm \leqq a = b \leqq 1,75nm$ et $2,80nm \leqq c \leqq 2,86nm$ et répondant à une formule qui, ramenée à une maille de la structure hexagonale, s'écrit

   $(uM_1q^+)\ (vH^+)[(SiO_2)_{96-x}\ (AlO_2)_x\ ]^{x-}\ \ (t\ H_2O)$

   avec dans cette formule x désignant un nombre tel que $3<x\leq24$ $M_1q^+$ désignant des cations d'au moins un métal de valence q choisi parmi les métaux alcalins et les métaux alcalinoterreux, et u, v, x et t étant des nombres tels que $t \geqq O$ selon le degré d'hydratation de la zéolithe, $t = O$ pour une zéolithe complètement anhydre, et que $qu + v \geqq x$, ledit catalyseur se caractérisant en ce que le nombre v de cations $H^+$ dans la formule de la zéolithe est tel que $0,5\ x \leqq v \leqq x$.

2. Catalyseur selon la revendication 1, caractérisé en ce que les proportions y% du liant inorganique et z% de la zéolithe sont telles que $4 \leqq y \leqq 96$ et $4 \leqq z \leqq 96$ avec $y + z = 100$.

3. Catalyseur selon la revendication 1 ou 2, caractérisé en ce que le nombre v de cations $H^+$ dans la formule de la zéolithe est tel que $0,8\ x \leqq v \leqq x$.

4. Catalyseur selon l'une des revendications 1 à 3, caractérisé en ce que dans la formule de la zéolithe $M_1q^+$ représente des cations d'au moins un élément choisi parmi Na, K, Li, Cs, Ca, Sr et Ba.

5. Catalyseur selon l'une des revendications 1 à 4, caractérisé en ce que la zéolithe présente dans le catalyseur possède une surface spécifique, déterminée par adsorption d'azote selon la méthode B.E.T. simplifiée à un point (norme NF X 11-622), comprise entre $100m^2/g$ et $1000m^2/g$ et de préférence entre $300m^2/g$ et $900m^2/g$.

**6.** Catalyseur selon l'une des revendications 1 à 5, caractérisé en ce qu'il renferme en plus du liant et de la zéolithe jusqu'à 15% et de préférence jusqu'à 10%, en poids de l'ensemble liant et zéolithe, d'au moins un métal catalytiquement actif, présent à l'état élémentaire ou sous la forme d'un composé métallique.

**7.** Catalyseur selon la revendication 6, caractérisé en ce que ledit métal catalytiquement actif est choisi parmi les métaux nobles platine, palladium, rhodium et iridium, les métaux de transition non nobles Ni, Co, W et Mo et le métal gallium.

**8.** Catalyseur selon l'une des revendications 1 à 7, caractérisé en ce que la zéolithe présente, après calcination à 600 °C pendant 4 heures, un diagramme de diffraction des rayons X comparable à celui du tableau I donné dans la description.

**9.** Catalyseur selon l'une des revendications 1 à 8, caractérisé en ce que le liant inorganique consiste en un ou plusieurs oxydes réfractaires choisis parmi alumine, silice, argile, silice/alumine et magnésie.

**10.** Application du catalyseur selon l'une des revendications 1 à 9, comme promoteur des réactions de catalyse acide réalisées sur des charges hydrocarbonées.

**11.** Application selon la revendication 10, caractérisée en ce que la réaction de catalyse acide est un craquage catalytique, notamment un craquage catalytique de distillats sous vide ou de résidus en essence, en opérant en particulier en lit fluidisé ou en lit mobile à des températures allant, par exemple, de 450 °C à 650 °C.

**12.** Application selon la revendication 10, caractérisée en ce que la réaction de catalyse acide est un hydrocraquage catalytique, notamment un hydrocraquage de distillats sous vide en gazole, en opérant à des températures supérieures à 250 °C, en particulier entre 350 °C et 500 °C, sous une pression supérieure à 15 bars, avantageusement supérieure à 30 bars, et avec une vitesse volumique horaire comprise entre 0,2 et 10.

**13.** Application selon la revendication 10, caractérisée en ce que la réaction de catalyse acide est une réaction d'isomérisation, en particulier isomérisation des hydrocarbures alcoylaromatiques tels que les xylènes.

**14.** Application selon la revendication 10, caractérisée en ce que la réaction de catalyse acide est une alkylation d'hydrocarbures aromatiques, notamment benzène, par les oléfines.

**Claims**

**1.** Catalyst containing, by weight, y% of an inorganic binder and z% of a zeolite related to faujasite and having a structure of hexagonal symmetry, y and z being numbers such that $1 \leqq y \leqq 99$ and $1 \leqq z \leqq 99$ with $y + z = 100$, the zeolite having parameters a, b and c of its hexagonal mesh such that $1.72 \text{ nm} \leqq a = b \leqq 1.75 \text{ nm}$ and $2.80 \leqq c \leqq 2.86 \text{ nm}$ and corresponding to a formula which, applied to a mesh of the hexagonal structure, is written as:

$$(uM_1 q^+)\ (vH^+)\ [(SiO_2)_{96-x}\ (AlO_2)_x]^{x-}\ (t\ H_2O)$$

with, in this formula, x designating a number such that $3<x\leqq 24$, $M_1{}^{q+}$ designating cations of at least one metal of valency q selected from among alkali metals and alkaline-earth metals, and u, v, x and t being numbers such that $t \geqq O$ according to the degree of hydration of the zeolite, $t = O$ for a completely anhydrous zeolite, and such that $qu + v \geqq x$, the catalyst being characterised in that the number v of cations $H^+$ in the formula of the zeolite is such that $0.5\ x \leqq v \leqq x$.

**2.** Catalyst according to Claim 1, characterised in that the proportions y% of the inorganic binder and z% of zeolite are such that $4 \leqq y \leqq 96$ and $4 \leqq z \leqq 96$ with $y + z = 100$.

**3.** Catalyst according to Claim 1 or 2, characterised in that the number v of cations $H^+$ in the zeolite formula is such that $0.8\ x \leqq v \leqq x$.

4. Catalyst according to any one of Claims 1 to 3, characterised in that in the zeolite formula $M_1^{q+}$ represents cations of at least one element selected from Na, K, Li, Cs, Ca, Sr and Ba.

5. Catalyst according to any one of Claims 1 to 4, characterised in that the zeolite present in the catalyst has a specific surface area, determined by adsorption of nitrogen according to the simplified one point B.E.T. method (NF standard X 11-622), between $100m^2/g$ and $1000m^2/g$ and preferably between $300m^2/g$ and $900m^2/g$.

6. Catalyst according to any one of Claims 1 to 5, characterised in that it contains in addition to the binder and zeolite up to 15% and preferably up to 10%, relative to the weight of the binder and zeolite together, of at least one catalytically active metal, present in the elementary state or in the form of a metal compound.

7. Catalyst according to Claim 6, characterised in that the catalytically active metal is selected from among the precious metals platinum, palladium, rhodium and iridium, non-noble transition metals Ni, Co, W and Mo and the metal gallium.

8. Catalyst according to any one of Claims 1 to 7, characterised in that, after calcination at 600 °C for 4 hours, the zeolite displays an X-ray diffraction diagram comparable to that of table I in the description.

9. Catalyst according to any one of Claims 1 to 8, characterised in that the inorganic binder consists of one or more refractory oxides selected from alumina , silica, clay, silica/alumina and magnesia.

10. Application of the catalyst according to any one of Claims 1 to 9, as promoter of acid catalysis reactions performed on hydrocarbon charges.

11. Application according to Claim 10, characterised in that the acid catalysis reaction is catalytic cracking, in particular catalytic cracking of vacuum distillates or of residues to form petrol, operating in particular in a fluidised bed or a moving bed at temperatures ranging from 450 °C to 650 °C for example.

12. Application according to Claim 10, characterised in that the acid catalysis reaction is catalytic hydrocracking, in particular hydrocracking of vacuum distillates to form gas oil, operating at temperatures of more than 250 °C, in particular between 350 °C and 500 °C, at a pressure of more than 15 bar, advantageously of more than 30 bar, and with an hourly volume velocity of between 0.2 and 10.

13. Application according to Claim 10, characterised in that the acid catalysis reaction is an isomerisation reaction, in particular isomerisation of the alkylaromatic hydrocarbons such as xylenes.

14. Application according to Claim 10, characterised in that the acid catalysis reaction is an alkylation of aromatic hydrocarbons, in particular benzene, by olefins.

**Patentansprüche**

1. Katalysator, der bezogen auf das Gewicht, y% eines anorganischen Bindemittels und z% eines Zeolithen mit einer Struktur von hexagonaler Symmetrie, die mit der Struktur des Faujasits verwandt ist, enthält, wobei y und z den Zahlenwerten $1 \leqq y \leqq 99$ und $1 \leqq z \leqq 99$ bei $y + z = 100$ entsprechen, und die Parameter a, b und c der hexagonalen Elementarzelle des Zeolithen die Werte $1{,}72nm \leqq a = b \leqq 1{,}75nm$ und $2{,}80nm \leqq c \leqq 2{,}86$ nm aufweisen und dieser einer Formel entspricht, die sich, zurückgeführt auf eine Elementarzelle von hexagonaler Struktur, mit $(uM_1q^+)$ $(vH^+)$ $[(SiO_2)_{96-x} (AlO_2)_x]^{x-}$ $(t\ H_2O)$ beschreiben läßt, wobei in dieser Formel x einem Zahlenwert $3<x\leqq24$ entspricht, $M_1q^+$ Kationen wenigstens eines Metalls mit der Wertigkeit q, ausgewählt unter Alkali- und Erdalkalimetallen, bedeutet, und u, v, x, und t den Zahlenwerten $t\geqq0$ je nach dem Hydratisierungsgrad des Zeolithen, t = o bei einem vollständing wasserfreien Zeolithen, und $qu + v \geqq x$ entsprechen, dadurch **gekennzeichnet**, daß die Zahl v der Kationen $H^+$ in der Formel des Zeolithen einem Wert $0\ 5x \leqq v \leqq x$ entspricht.

2. Katalysator nach Anspruch 1, **dadurch gekennzeichnet**, daß die Mengen an y% des anorganischen Bindemittels und z% des Zeolithen $4 \leqq y \leqq 96$ und $4 \leqq z \leqq 96$ bei $y + z = 100$ betragen.

3. Katalysator nach Anspruch 1 oder 2, **dadurch gekennzeichnet**, daß die Zahl v der Kationen $H^+$ in der Formel des Zeolithen dem Wert $0{,}8x \leqq v \leqq x$ entspricht.

4. Katalysator nach einem der Ansprüche 1 bis 3, **dadurch gekennzeichnet**, daß in der Formel des Zeolithen $M_1q^+$ Kationen wenigstens eines Elements darstellt, ausgewählt unter Na, K, Li, Cs, Ca, Sr und Ba.

5. Katalysator nach einem der Ansprüche 1 bis 4, **dadurch gekennzeichnet**, daß der im Katalysator vorliegende Zeolith eine spezifische Oberfläche, ermittelt durch Stickstoffabsorption nach der vereinfachten BET-Einpunktmethode (Norm NF X 11-622),zwischen 100 $m^2/g$ und 1000 $m^2/g$ und vorzugsweise zwischen 300 $m^2/g$ und 900 $m^2/g$ besitzt.

6. Katalysator nach einem der Ansprüche 1 bis 5, **dadurch gekennzeichnet**, daß er außer dem Bindemittel und dem Zeolithen bis zu 15% und vorzugsweise bis zu 10%, bezogen auf das Gesamtgewicht von Bindemittel und Zeolith, wenigstens eines katalytisch aktiven Metalls in elementarem Zustand oder in Form einer Metallverbindung enthält.

7. Katalysator nach Anspruch 6, **dadurch gekennzeichnet**, daß das katalytisch aktive Metall ausgewählt ist unter den Edelmetallen Platin, Palladium, Rhodium und Iridium, den unedlen Übergangsmetallen Ni, Co, W und Mo und dem Metall Gallium.

8. Katalysator nach einem der Ansprüche 1 bis 7, **dadurch gekennzeichnet**, daß der Zeolith nach Glühen bei 600 °C während vier Stunden ein Röntgenbeugungsdiagramm aufweist, das mit dem in Tabelle 1 des Beschreibungstextes vergleichbar ist.

9. Katalysator nach einem der Ansprüche 1 bis 8, **dadurch gekennzeichnet**, daß das anorganische Bindemittel aus einem oder mehreren feuerfesten Oxiden besteht, ausgewählt unter Tonerde,Kieselerde, Ton, Kieselerde/Tonerde und Magnesia.

10. Verwendung des Katalysators nach einem der Ansprüche 1 bis 9 als Beschleuniger für an Kohlenwasserztofffraktionen durchgeführte Säuerkatalysereaktionen.

11. Verwendung nach Anspruch 10, **dadurch gekennzeichnet**, daß die Säurekatalysereaktion ein Katkrackprozeß ist, insbesondere ein Katkracking von Vakuumdestillaten oder von Rückständen zu Benzin, insbesondere in einem Wirbel- bzw. Fließbett, bei Temperaturen, z.B. zwischen 450 bis 650 °C.

12. Verwendung nach Anspruch 10, **dadurch gekennzeichnet**, daß die Säurekatalysereaktion ein katalytisches Hydrokracking, insbesondere ein Hydrocracking von Vakuumdestillaten zu Dieselkraftstoff bei Temperaturen von über 250 °C, insbesondere zwischen 350 und 500 °C, unter einem Druck von über 15 bar, vorteilhaftenweise von über 30 bar, und bei einer Raumgeschwindigkeit pro Stunde von 0,2 bis 10 ist.

13. Verwendung nach Anspruch 10, **dadurch gekennzeichnet**, daß die Säurekatalysereaktion eine Reaktion der Isomerisierung, insbesondere von alkylaromatischen Kohlenwasserstoffen wie Xylolen ist.

14. Verwendung nach Anspruch 10, **dadurch gekennzeichnet**, daß die Säurekatalysereaktion eine Alkylierung von aromatischen Kohlenwasserstoffen, insbesondere von Benzol, durch Olefine ist.